# EUROPEAN PATENT APPLICATION

(11) **EP 1 096 246 A1**
(43) Date of publication of application: **02.05.2001**
(21) Application number: 99870227.8
(22) Date of filing: 29.10.1999
(51) Int. Cl.: G01N 19/02, G01N 33/44

(54) **Test method and apparatus for assessing a tendency to squeak of an assembly**

(71) Applicant: RECTICEL, 1200 Brussels (BE)
(72) Inventor: Rombaut, Hubert, 9230 Wetteren (BE); Coppens, Pierre, 9230 Wetteren (BE); Huysentruyt, Philippe, 9230 Wetteren (BE); Mladenovic, Vladimir c/o Université P. & M. Curie, 75007 Paris (FR)
(74) Representative: Van Reet, Joseph

(57) **Abstract**

The invention relates to a test method and apparatus for assessing whether an assembly, which comprises as components at least a gasket (1) and a substrate (2) against which the gasket is pressed, has a tendency to squeak when the gasket (1) is moved relative to the substrate (2). For assessing said tendency, use is made of at least one accelerometer to detect whether vibrations are generated in the assembly when the gasket is moved relative to the substrate, in particular by means of an oscillator (15). In contrast to the existing methods which are based on acoustic measurements, the test method according to the invention is not influenced by background noises so that no acoustic insulation is required.

## Description

The present invention relates to a test method for assessing whether an assembly, which comprises as components at least a gasket and a substrate against which the gasket is pressed, has a tendency to squeak when the gasket is moved relative to the substrate, in particular when the assembly is subjected to vibrations.

The problem of squeaking arises especially in the automotive industry. It has often been observed that due to vibrations, for example when a car is traversing a bumpy road, audible noises are produced by the car windows, especially when the car is still quite new. These noises are caused by relative motions of the car windows, which are encapsulated with a gasket, and the car body. They are more particularly generated when, due to the relative motions of car body and windows, the window encapsulation gaskets rub against the painted sheet metal of the car body.

In order to obviate the problem of squeaking, a low friction tape or a flocking can be applied onto the window encapsulation gasket before mounting the encapsulated window in the car body. It will be clear that such solutions show a number of disadvantages, including the fact that additional materials are needed and that additional production time is required to apply them. An easier and better way to avoid squeaking would be the use of gaskets which are less prone to generating the squeak noises. In this respect US-A-5 770 674 discloses the use of particular polyurethane compositions for producing gaskets which would prevent squeaks.

US-A-5 770 674 also discloses an apparatus and a method for testing the squeak intensity of test plaques moulded from the polyurethane compositions. The disclosed apparatus and method enable to assess the tendency to squeak of a material without having to encapsulate a window therewith, mount the window in a car body and doing practical road tests. The test apparatus disclosed in US-A-5 770 674 comprises means for holding a piece of the substrate, i.e. a flat panel of painted sheet metal, into contact with the test plaque and an oscillator for moving the test plaque in a sinuous motion against the surface of the substrate. The holding means are arranged in an acoustically insulated acoustic box whilst the oscillator is arranged outside this box. Inside the box is disposed a microphone which picks up any squeaking noises produced when the test plaque is rubbed against the surface of the substrate. The output of the microphone is converted to digital values which are processed in a computer. This computer more particularly collects the noise data for 60 seconds, filters out low frequency noise below 100 Hz, calculates the power spectrum and integrates the total power intensity of the signal. This total power intensity is used as an indication of the squeak intensity.

A drawback of the test apparatus and method disclosed in US-A-5 770 674 is that the acoustical measurements require an effective acoustical insulation of the experimental set up and that any background noises which are still present in the acoustic box will interfere with the measurements of the squeaking noises.

An object of the present invention is now to provide a test method which enables to assess whether an assembly, comprising as components at least a gasket and a substrate against which the gasket is pressed, has a tendency to squeak when the gasket is moved relative to the substrate, but which test method is not influenced by background noises so that no acoustical insulation is required and so that the test method can thus be performed much easier.

To this end, the test method according to the invention is characterised in that that for assessing said tendency, use is made of at least one accelerometer to detect whether vibrations are generated in the assembly when the gasket is moved relative to the substrate. The accelerometer is more particularly used to measure the acceleration of at least one component of the assembly as a function of time to determine whether vibrations are generated in the assembly when the gasket is moved relative to the substrate, especially vibrations with a frequency in the audible range. These vibrations are called hereinafter high frequency vibrations or second vibrations.

According to the invention it has been found that to assess the squeaking behaviour of an assembly comprising a gasket pressed against a substrate one does not have to measure the squeaking noises themselves but the squeaking behaviour can be measured directly on one or more components of the assembly, namely by measuring the vibrations thereof by means of an accelerometer. It is clear that such direct measurements are not influenced by background noises. In this way, it is for example possible to determine the occurrence of squeaking noises in a riding car by placing an accelerometer against the car window, even when the squeaking noises have an intensity lower than the intensity of the background noises in the car, for example smaller than 45 dB, or in other words even in case the squeaking noises cannot be heard. According to the invention, it has been found during experiments carried out in a riding car that the occurrence of audible squeaking noises is linked to the occurrence of high frequency vibrations in the window. More particularly, it is put forward by the invention that squeaking noises are the result of the high frequency vibrations generated by the gasket rubbing over the car body so that the absence of such vibrations can be correlated with no tendency to squeak.

A preferred embodiment of the method according to the invention comprises the steps of:
- pressing at least a piece of the gasket against at least a piece of the substrate;
- imposing a first vibration on at least one assembly component to obtain a relative motion between the gasket and the substrate;
- measuring the acceleration of at least one assembly component as a function of time during said first vibration; and
- generating an output signal which indicates said tendency to squeak in the case that, based on the measured acceleration values, said assembly component is subjected to at least one second vibration, having a frequency higher than the frequency of the first vibration.

The output signal may comprise a graph showing the acceleration values of at least said second vibration as a function of time and/or a value indicating an amplitude of the acceleration of said second vibration. On the graph of the acceleration values it can immediately be seen whether second vibrations, having a frequency higher than the basic first vibration, are generated in the assembly and the amplitude or another value indicating the amplitude of these vibrations can also be read or calculated from that graph. In case such vibrations are generated, there is a tendency to squeak. However, when the amplitude or the acceleration amplitude indicating value is smaller than a threshold acceleration amplitude indicating value pre-established for the second vibration the tendency to squeak can be acceptably low whereas an acceleration amplitude indicating value greater than said pre-established threshold acceleration amplitude indicating value indicates, on the other hand, an unacceptably high tendency to squeak. When the assembly is destined for a predetermined application in an environment with background noises, the said threshold acceleration amplitude value can be pre-established experimentally by taking reference samples of gaskets which squeak in said predetermined application at a level lower than the level of said background noises, more particularly for automotive applications at a level lower than 45 dB, by subjecting these reference samples to the test method wherein said acceleration amplitude indicating value is determined and by selecting as said threshold acceleration amplitude indicating value, a value equal to or smaller than the largest acceleration amplitude indicating value of the second vibrations determined for the reference samples.

The present invention also relates to an apparatus for performing the above described test method, i.e. an apparatus for assessing whether an assembly, which comprises as components at least a gasket and a substrate against which the gasket is pressed, has a tendency to squeak when the gasket is moved relative to the substrate, which apparatus comprises first means for holding at least a piece of the gasket and second means for holding at least a piece of the substrate, the first and second holding means being arranged to press the piece of the gasket against the piece of the substrate, and means for moving the gasket relative to the substrate when being pressed against one another. The test apparatus according to the invention is characterised in that it further comprises at least one accelerometer for measuring the acceleration of at least one assembly component as a function of time during said first vibration.

Other particularities and advantages of the invention will become apparent from the following description of some particular embodiments of the test method and the test apparatus according to the present invention. The reference numerals used in this description relate to the annexed drawings wherein:
Figure 1 shows a side elevational view on a test apparatus according to the invention with partial cut-aways;

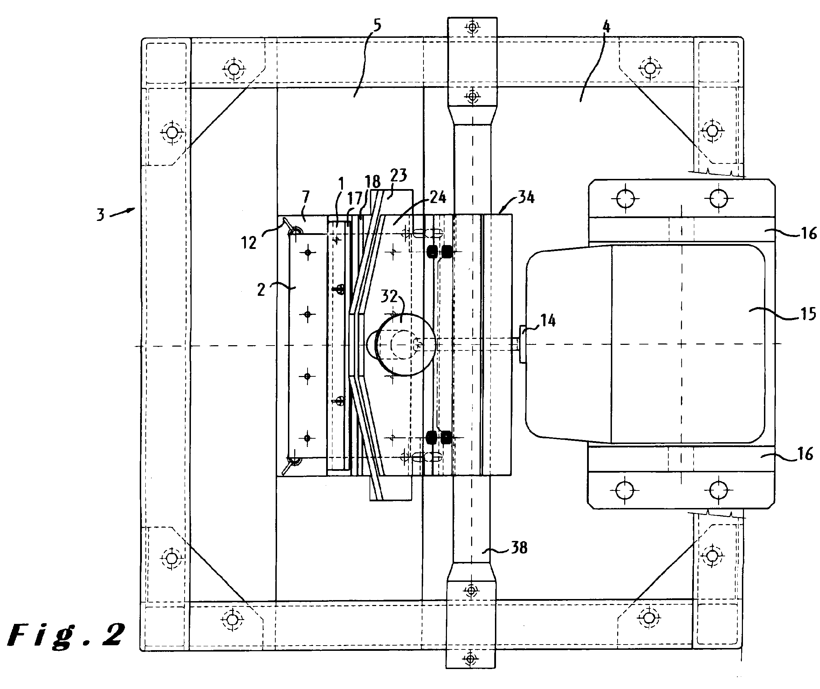

Figure 2 shows a top plan view on the test apparatus shown in Figure 1;
Figure 3 shows, on a larger scale, an exploded view of the components of the gasket/glass holding means of the test apparatus shown in Figures 1 and 2;
Figure 4 shows, on a still larger scale, an exploded view of the glass clamp shown in Figure 3;
Figure 5 shows, on a larger scale, a perspective view on the test unit of the test apparatus shown in Figures 1 and 2;
Figure 6 shows a side elevational view analogous to Figure 1 but showing the test apparatus with the weighing unit instead of with the test unit;
Figure 7 shows a diagram of electronic components suited for carrying out the method according to the invention with the apparatus shown in the previous figures; and
Figures 8a and 8b show a graph of the acceleration values measured on the glass plate and respectively on the substrate plate in the apparatus shown in Figures 1 and 2 in function of the time.

In the test method according to the invention there is assessed whether an assembly, which comprises as components at least a gasket 1 and a substrate 2 against which the gasket 1 is pressed, has a tendency to squeak when the gasket is moved relative to the substrate. This relative movement may occur when the assembly is subjected to low frequency vibrations. A typical example is a window encapsulating gasket arranged around a window of an automobile and mounted in a car body. In this case the painted sheet metal of the car body is the substrate. It will be clear that the gasket can also be applied around other elements or that it may be an integral part of such elements but the present description will now be given with specific reference to window encapsulation gaskets.

When driving over bumpy roads so that the car is subjected to low frequency vibrations having a frequency which is usually lower than 100 Hz and most frequently lower than 20 Hz, squeaking noises can be produced as a result of a relative motion between the window or the window encapsulating gasket and the car body. It has been found that this relative displacement is in the order of magnitude of 0.1 to 2.0 mm. The largest displacements occur when the window is only mounted for some hours in the car body and they decrease as the gasket becomes older.

In practice, the tendency of the combination of a gasket and a substrate to squeak can be assessed by driving the car having a window encapsulated with the gasket mounted therein over a road and by listening to or recording any squeak noises. As already set forth hereabove, such a method has the drawback that low squeaking levels cannot be heard or detected and that it is quite laborious and expensive to encapsulate each time a window with the gasket which is to be tested, in particular if several designs are to be tested each requiring different moulds for making the gasket. In practice, entire cars are sometimes artificially vibrated in special, acoustically insulated chambers to eliminate any background noises but this is certainly a very expensive procedure.

In the method according to the invention, the influence of background noises is eliminated in a quite easy way namely by using an accelerometer to detect whether vibrations are generated in the gasket-substrate assembly when the gasket is moved relative to the substrate, in particular when the assembly is subjected to said low frequency vibrations. The vibrations in the assembly can be measured by placing the accelerometer onto any of the components of the assembly. Preferably the accelerometer is placed onto the encapsulated window, more particularly in such a manner that the acceleration of the window is measured in a direction substantially perpendicular to the surface thereof although the acceleration can also be measured in other directions. In a direction perpendicular to the surface of the window surface, the acceleration of the generated vibrations is however the largest and is thus easier to measure.

According to the invention it has been found that the observed squeaking noises are the result of mechanical vibrations having a relatively high frequency, more particularly in the audible range, generated by vibrations of a lower frequency to which the assembly, in this case the car, is subjected and further that these relatively high frequency vibrations can be measured, notwithstanding the fact that the entire assembly is vibrating at a lower frequency but at a very much larger amplitude than the displacement amplitude of the high frequency vibrations which cause the squeaking noises. It can for example be calculated theoretically that for a frequency of 1 kHz, a squeaking level of 80 dB in a car corresponds to an amplitude of the displacement of the windows in a direction perpendicular to the surface thereof of about 0.1 µm whereas the entire window may vibrate in that same direction at an amplitude of about 1 mm with respect to the car body at some low frequency, for example at 10 Hz. A vibration with such low amplitude compared to the amplitude of the basic vibration by which it is generated and onto which it is superimposed cannot be detected in any normal or standard way. An essential feature of the method according to the invention is, however, that not the actual displacements are measured but the varying accelerations during the low and possible high frequency vibrations. The basic vibrations of a relatively low frequency to which the assembly is subjected will be called hereinafter first vibrations and the vibrations of a higher frequency in the audible range generated thereby will be called second vibrations. These second vibrations have generally a frequency in the range of 100 Hz to 20 kHz whereas the first vibrations have generally a frequency below 100 Hz.

An important advantage of measuring accelerations instead of displacements is that due to the higher frequency of the second vibrations compared to the frequency of the basic first vibrations the amplitude of the accelerations of both vibrations are much more of the same order of magnitude. In fact, based on theory, the acceleration amplitude of a vibration is equal to 4Πf²ξₘₐₓ, wherein f = frequency of the vibration and ξₘₐₓ the displacement amplitude of the vibration. If the first vibration has a frequency of 10 Hz and if it produces a second squeaking vibration with a frequency of 1000 Hz, the ratio between the acceleration amplitudes is 10⁴ times smaller than the ratio between the displacement amplitudes. For the above example, the acceleration amplitudes of both vibrations would thus be in the same order of magnitude and are thus easily measurable with existing accelerometers.

In the method according to the invention, whether a gasket-substrate assembly has a tendency to squeak can be assessed in a car driving over a bumpy road or in a car which is artificially vibrated at a relatively low frequency, in particular of upto 100 Hz, by measuring the acceleration of at least one assembly component, preferably the encapsulated car window, as a function of time. The graph of the measured acceleration values can be used as an output signal which indicates a tendency to squeak in the case that it shows the presence of a second vibration which has a frequency higher than the frequency of the first vibration to which the car is subjected. Practical experiments have shown that, when squeaking noises could be heard in the vibrating car, second vibrations of a higher frequency were recorded with the accelerometer.

In order to enhance the accuracy of the test method, it is possible to filter the low frequency vibrations of the car out of the output signal. This can be done in particular by means of a second accelerometer which is placed on the car body. The acceleration values measured by this accelerometer can be detracted from the acceleration values of the window measured by the first accelerometer. In the thus obtained graph of acceleration differences, the appearance of second vibrations is usually clearer.

In the above description, the test method according to the invention has been described as being applied to window encapsulation gaskets which are already mounted in a car. However, the test method according to the invention is especially suited for being used in the design phase of a new gasket and/or substrate, more particularly for assessing or predicting the influence of the design of a gasket or the material thereof on the tendency to squeak in the later application without having to test the gasket-substrate combination in that actual application. Instead of having to make an entire gasket, only a small sample or piece of the gasket is made. When only the influence of the material of the gasket on the tendency to squeak is to be tested, the sample piece may simply consist of a plaque of the material to be tested. Of course when the influence of the design is to be tested, the sample piece has to have the same design as the actual gasket but may be of a much shorter length.

For assessing whether a gasket-substrate assembly has a tendency to squeak, the test piece of the gasket is pressed in an experimental set up against a sample piece of the substrate and the gasket is moved relative to the substrate. The relative movement of the gasket with respect to the substrate may be a unidirectional translational movement. However, preference is given to imposing a basic, first vibration on at least one component of the assembly to obtain the required relative motion between the gasket and the substrate. The sample piece of the substrate may consist of a painted sheet metal plate, i.e. a portion of the car body, but it may also consist of another material, in particular a synthetic material such as ABS or PVC, coated with the same paint so that it has the same effect on the squeaking behaviour of the gasket. The relative displacement between the substrate piece and the gasket piece is preferably in the order of magnitude of 0.1 to 10 mm and most preferably between 0.2 and 5 mm. Depending on the application, the basic first vibration can have a frequency of between 1 and 100 Hz, more particularly between 1 and 50 Hz and especially between 1 to 20 Hz. It should however be noted that higher frequencies can also be used The frequency of this first vibration may be constant or may be varied during the measurements either according to a fixed or a random pattern or also according to a pattern recorded in the real situation of the application for which the assembly is destined.

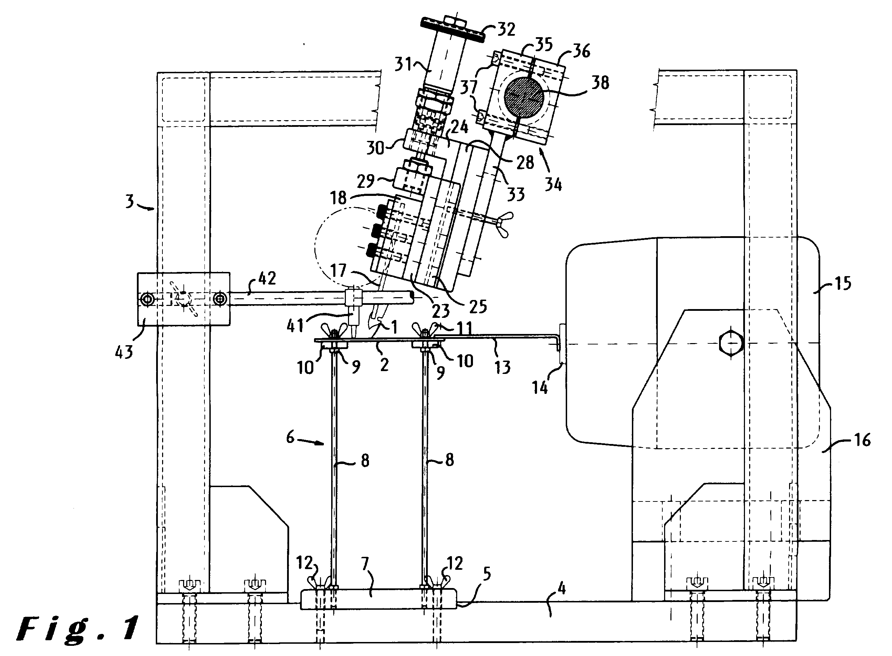

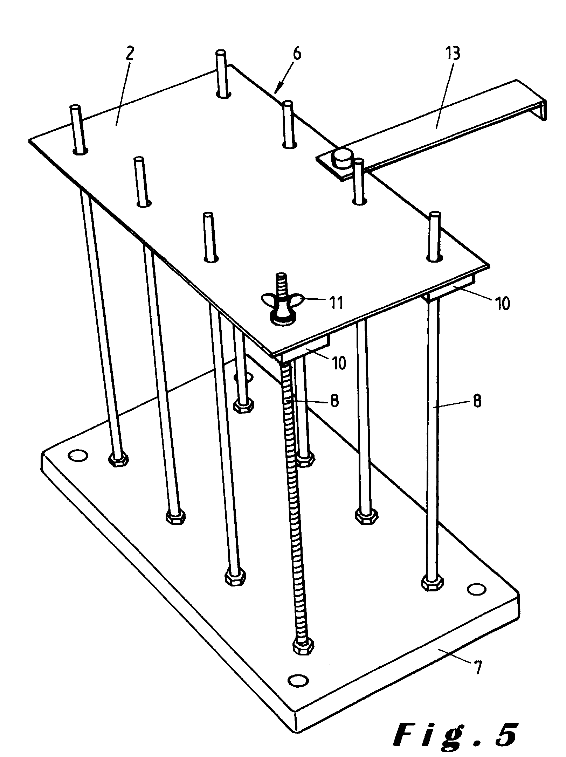

An example of a test apparatus which can be used for performing the test method according to the invention is shown in the figures. This apparatus comprises a rigid frame 3 mounted on a relatively thick and solid base plate 4. The base plate 4 is provided with a guiding groove 5 wherein a test unit 6, shown more into detail in Figure 5, can be fixed. This test unit 6 comprises a sliding plate 7 having eight treaded rods 8 screwed in upright position therein. On top of the rods 8, nuts 9 are provided on the desired height which bear two parallel laths 10. The piece of the substrate 2, which is provided with holes corresponding to the rods 8, is positioned on the laths 10 and fixed by means of wing screws 11. As shown in Figure 1, the sliding plate 7 of the test unit 6 is rigidly secured by means of wing screws 12 in the guiding groove 5 in the base plate 4 of the test apparatus. On top of the test unit, the piece of substrate 2 is connected by means of a drive connector 13 to the oscillating head 14 of a mechanical oscillator or shaker 15 mounted onto uprights 16 fixed to the base plate 4. Due to the flexibility of the treaded rods 8, the piece of substrate 2 can be oscillated in a substantial horizontal direction with the desired amplitude and frequency by means of the oscillator 15.

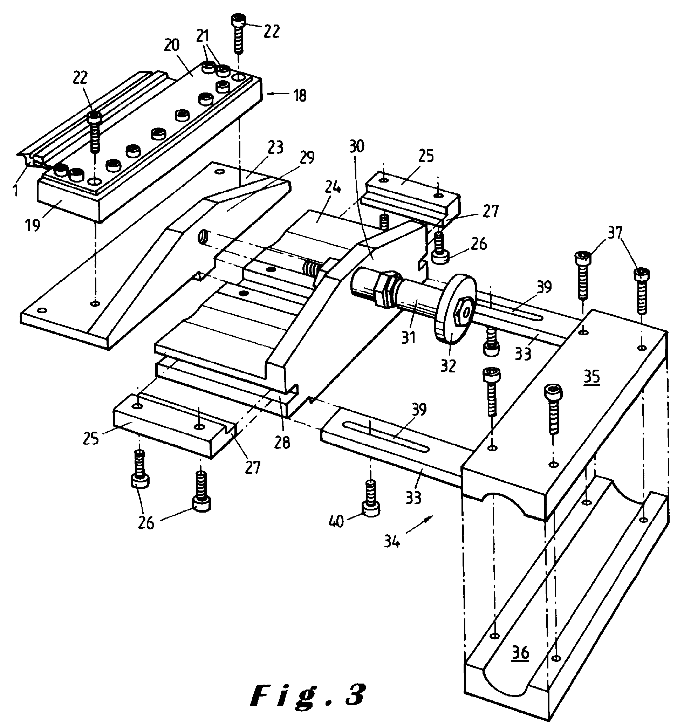

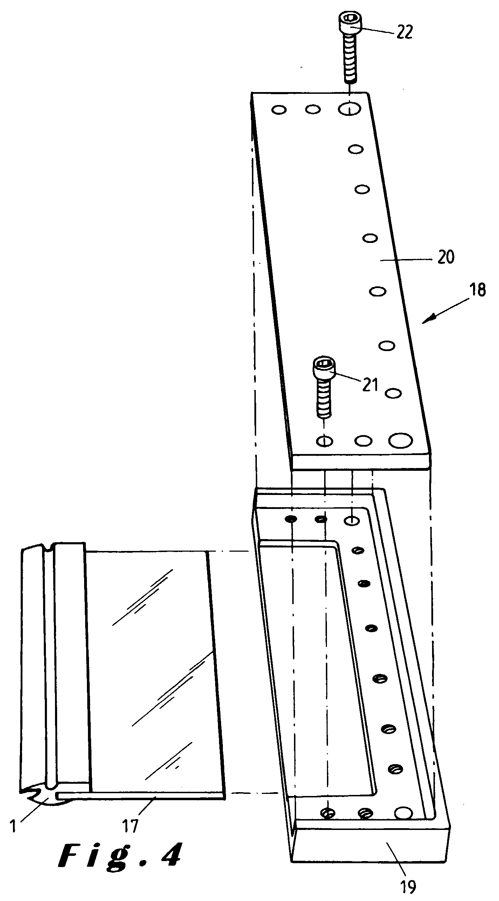

For assessing whether an assembly which comprises the substrate 2 and a gasket 1 has a tendency to squeak when these components are moved relative to one another, a piece of the gasket 1 is pressed on top of the piece of substrate 2. In Figure 1, the gasket 1 is applied around the edge of a plate 17, more particularly a glass plate, fixed in holding means, the components of which are shown in detail in Figures 3 and 4.

The glass plate 17 is first of all clamped in a clamp 18 between a bottom plate 19, showing a recess corresponding to the shape of the glass plate 17, and a top plate 20. Both plates 19 and 20 are pressed together by means of bolts 21.

As shown in Figure 3, the glass clamp 18 is fixed by means of two bolts 22 to a first carriage 23. This first carriage 23 can slide on a second carriage 24 to which it is slideably fixed through the intermediary of clamping laths 25. These laths 25 are rigidly screwed to the back of the first carriage 23 by means of bolts 26 and show each a lateral projection 27 arranged to slideably engage a groove 28 in both sides of the second carriage 24. Both the first 23 and the second carriage 24 show a flange 29 and 30 respectively. A nonius 31 extends through the flange 30 in the second carriage 24 into the flange 29 in the first carriage 23 and is arranged to adjust the mutual position between the two carriages 23, 24 by rotating the wheel 32 either clockwise or anticlockwise. Since such a nonius is known per se, its internal structure need not to be described further into detail.

The second carriage 24 is mounted onto projecting legs 33 of rotating clamp 34, consisting of two parts 35, 36 clamped by means of bolts 37 around a cylindrical shaft 38 which is rigidly fixed to the top of the frame 3. In the legs 33, two slots 39 are made through which bolts 40 are applied for fixing the second carriage 24 to the rotating clamp 34. It will be clear that the slots 39 in the legs 33 of the rotating clamp 34 enable a rough adjustment of the height of the gasket piece 1 on top of the substrate piece 2 and that the angular position of the gasket piece 1 with respect to the substrate piece 2 can be adjusted by clamping the rotating clamp 34 under the desired angle onto the shaft 38.

The test apparatus shown in the figures further comprises an altitude meter 41 mounted through the intermediary of a support shaft 42 and clamps 43 between two comer uprights of the frame 3. This altitude meter 41 enables to measure the level of the top surface of the substrate piece 2 when sliding the test unit 6 through the groove 5 in the base plate 4 underneath the meter 41.

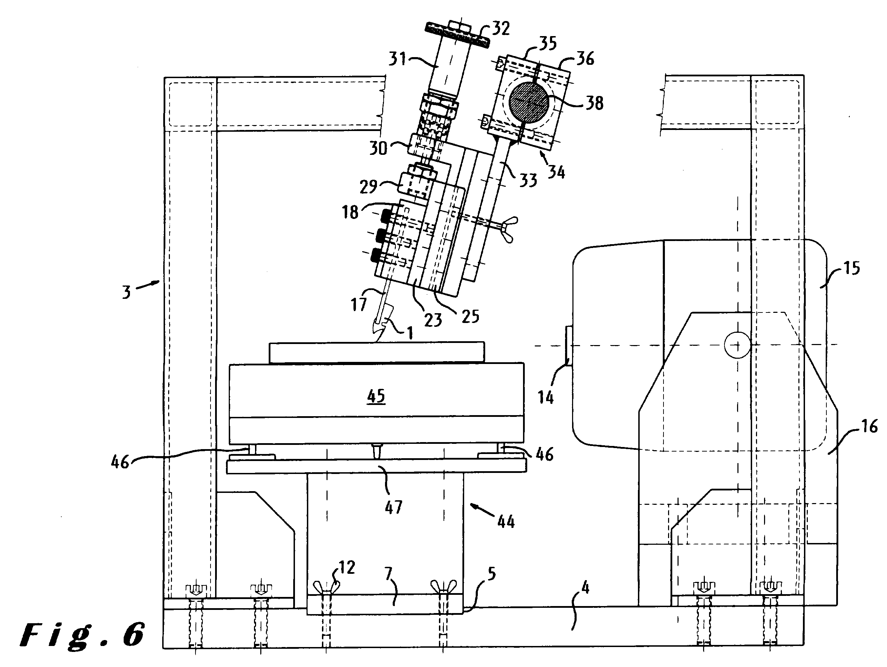

As shown in Figure 6, a weighing unit 44, provided with a similar sliding plate 7 as the test unit 6, can be slid in the groove 5 instead of the test unit 6. This weighing unit 44 comprises a balance 45 resting by means of height adjustable feet 46 onto a support plate 47. When positioning the weighing unit 44 underneath the altitude meter 41, the top surface of the balance 45 can be arranged on the same height as the top surface of the test unit 6. By sliding the weighing unit subsequently underneath the gasket piece 1, the pressure exerted by this piece 1 onto the balance 45 can be measured which pressure is equal to the pressure exerted by the gasket piece 1 onto the substrate piece 2 of the test unit 6. The weighing unit thus enables to measure or to adjust the pressure which is exerted by the gasket onto the substrate. In this way, the squeaking tendency can be determined for example for a standard pressure. This standard pressure is indeed not always the same as a standard distance from the substrate since, for a same mutual distance, the pressure may vary depending on the softness of the gasket and/or of the design thereof.

The test apparatus according to the present invention finally comprises at least one accelerometer 48, 48' which can be placed or glued against any element of the assembly which is to be tested, in particular against the glass plate 17, the gasket piece 1 or the substrate piece 2. The accelerometer can be positioned in different directions in order to measure the acceleration of the respective assembly component in the desired direction.

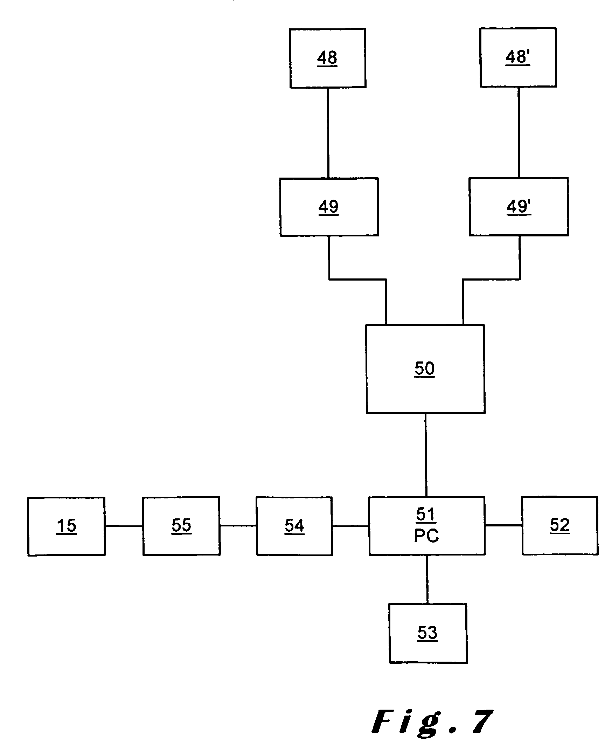

Figure 7 shows schematically a diagram of the electronics which can be coupled to the accelerometer. The accelerometers 48, 48' are more particularly coupled over an amplifier/power supply 49 and 49' respectively to an oscilloscope 50. In this oscilloscope 50, the amplified signals from the accelerometers 48, 48' are converted from analogue to digital values. The oscilloscope 50 further comprises a screen onto which the measured acceleration values can be displayed as a graph in function of the time. The oscilloscope 50 also enables to perform a Fourrier transformation onto the measured values and to display these values as a function of the frequency of the measured vibration. It further allows to filter out the lower frequencies, for example frequencies lower than 100 Hz. A more detailed description of the oscilloscope should not be given since oscilloscopes suitable for the described functions are readily available on the market. In the scheme of Figure 7, the oscilloscope 50 is connected to a personal computer or PC 51 wherein the digital values from the oscilloscope 50 can be processed further and shown on a screen 52 or printed on a printer 53. The PC 51 can further be used to control, over a signal generator 54 and an amplifier 55, the oscillations of the oscillator 15, more particularly the stroke length or amplitude and the frequency thereof.

With the above described test apparatus, a test method according to the invention for assessing whether a gasket-substrate assembly has a tendency to squeak can be performed quite easily. First of all, a piece of substrate 2 is prepared and a piece of gasket 1 which is applied around the edge of the glass plate 17. This can be done by gluing or the gasket 1 can also be moulded in situ around this edge. The encapsulated glass plate 17 is fixed in the glass clamp 18 and pressed with the gasket piece 1 against the substrate piece 2.

Subsequently, an accelerometer 48 is glued or otherwise fixed to at least one component of the assembly. In the apparatus shown in the drawings, an accelerometer is preferably glued onto the glass plate 17, more particularly in such a manner that accelerations in a direction perpendicular to the surface of this plate 17 are measured. The oscillator 15 is then started to impose a first vibration on the substrate piece 2 to obtain a relative motion between the gasket 1 and the substrate 2.

Based on the acceleration values measured during the first vibration, an output signal is generated which indicates a tendency to squeak in case that the assembly component of which the acceleration is measured, i.e. the glass plate, is subjected to at least one second vibration, having a frequency higher than the first vibration. As explained hereabove, such a second vibration is caused by the rubbing action of the gasket on the substrate and causes the squeaking noises.

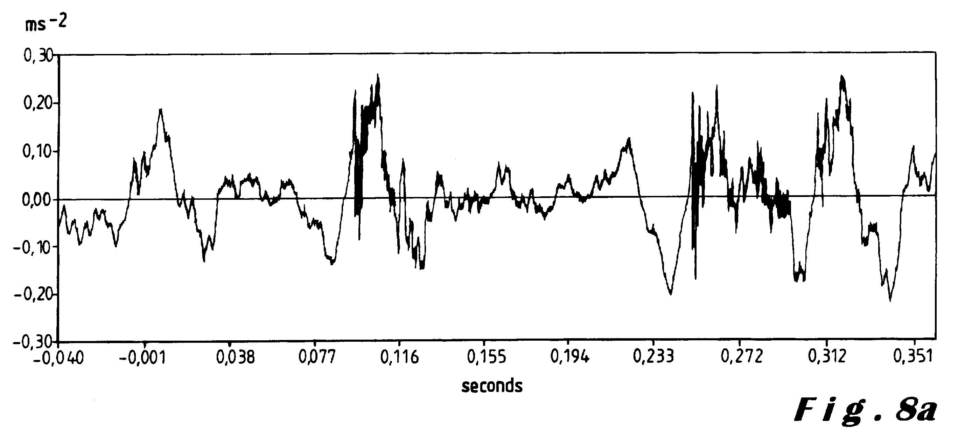

In a simple embodiment, the output signal comprises a graph showing the acceleration values of at least the second vibration as a function of time. An example of such a graph is shown in Figure 8a. In this graph, the second vibration is superimposed on the first vibration. The fact that such second vibrations, having a frequency higher than the first vibration, are generated, indicates that there is a tendency to squeak.

Although the second vibrations can be seen on the graph of Figure 8a, it may be preferred to provide a suitable filter in the oscilloscope 50 and/ or in the PC 51 to filter out the first vibration so that only the second vibrations would remain. When filtering the data in the PC, one could use for example the control data which have been transmitted by the PC to the oscillator 15. In the oscilloscope 50 the data can also be filtered. It is for example possible to filter out the lower frequencies, in particular the frequencies lower than 100 Hz. On the other hand, it is also possible to measure with the second accelerometer 48' the acceleration values of the substrate 2, in particular in the direction wherein the head 14 of the oscillator 15 moves, and to filter the signal of the first accelerometer 48 by detracting the values measured by the second accelerometer 48' therefrom. In this way, the first vibration is filtered out of the signal.

Instead or in addition to the above described graph, the output signal may also comprise a value indicating an amplitude of the acceleration of the second vibration. In this way, a further distinction can be made between gasket-substrate assemblies which have a high tendency to squeak and assemblies which have only a low tendency to squeak and which can thus still be acceptable in practice, for example since they only squeak at a level lower than the level of the usual background noises or since they produce only annoying squeaking noises in very unusual circumstances, for example when being subjected to exceptionally violent vibrations. In this method, an acceleration amplitude indicating value smaller than a threshold acceleration indicating value pre-established for the second vibration thus indicates an acceptable low tendency to squeak and an acceleration amplitude indicating value greater than said pre-established threshold acceleration amplitude indicating value an unacceptable high tendency to squeak. Since the threshold of hearing for the human ear depends on the frequency of the noise signal, the threshold acceleration amplitude indicating value may be determined for different frequencies, although between a frequency of about 800 to about 5000 Hz, the threshold of hearing remains substantially in the same order of magnitude.

In a preferred embodiment, the threshold acceleration amplitude indicating value is pre-established as a standard for a predetermined application of the assembly in an environment with background noises by taking reference samples of gaskets which squeak in said predetermined application at a level lower than the level of said background noises, more particularly for automotive applications at a level lower than 45 dB, by subjecting these reference samples to the test method wherein said acceleration amplitude indicating value is determined under standardized conditions and by selecting as said threshold acceleration amplitude indicating value a value equal to or smaller than the largest acceleration amplitude indicating value of the second vibrations determined for the reference samples.

The acceleration amplitude indicating value can be determined in different ways. First of all, it can be determined by determining the amplitudes of a number of successive peaks in the acceleration values of the second vibration and by selecting the maximum amplitude and/or by making an average of the determined amplitudes. This can either be done by the PC or it can be done manually starting from the graph of the measured acceleration values. Care should preferably be taken to eliminate the effect of the acceleration values resulting from the first vibration, for example by filtering this vibration out of the graph.

The acceleration amplitude indicating value can also be determined as an integrated surface area over a predetermined time period of the measured function between the acceleration of the second vibration and the time. In order to avoid that the positive acceleration values are compensated by the negative ones, only the positive or the negative parts of the graph should be integrated or the absolute value should be taken of all the acceleration values.

Before determining the acceleration amplitude indicating value for the second vibration, the measured values may be subjected, in particular in the oscilloscope 50, to a Fourrier transformation, more particularly to a fast Fourrier transformation (FFT). In this way, the amplitude of the acceleration can be seen for different frequencies. Starting from this transformed graph, further calculations can be made by the PC to obtain a value which is an indication of the amplitude of the acceleration amplitude of the second vibrations. Experiments have shown, that after a Fourrier transformation, it can clearly be seen whether the assembly is subjected to second vibrations. In one experiment, this second vibration had a main frequency around 1.4 kHz.

In the method according to the invention, the acceleration of the glass plate 17 is preferably measured. In order to achieve the greatest acceleration values for the second vibrations, this is preferably done in a direction forming an angle larger than 45° and more preferably larger than 75° with the surface of the glass plate 17. Instead of a glass plate 17, use could also be made of a plate of another material, for example a metal plate provided it shows the required rigidity for enabling to obtain any squeaking effect. In a preferred embodiment, like in the above described test apparatus, the first vibration is indeed preferably imposed on the substrate 2 in a direction forming an angle with the glass plate 17, in particular an angle larger than 45°. In this way, the vibrations of the glass plate 17 are less hampered by the pressure exerted thereon by the substrate 2 so that the second vibrations can be measured more easily.

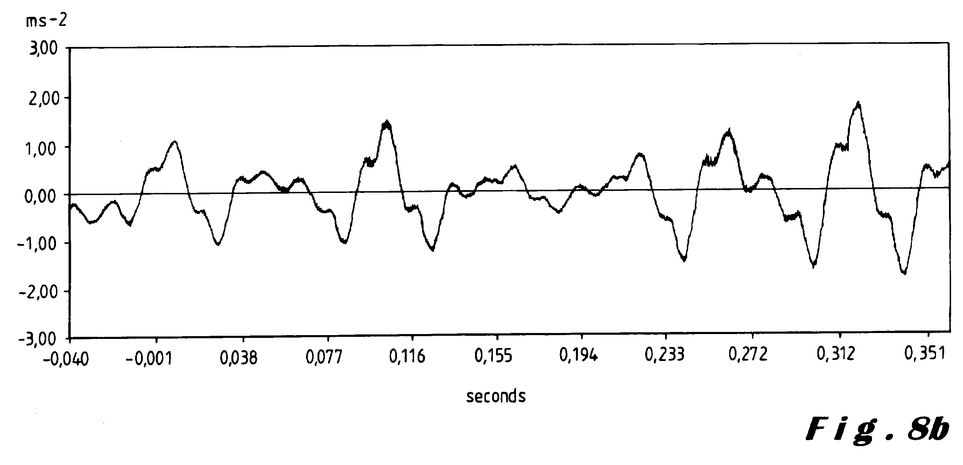

In a further embodiment of the invention, the acceleration of the substrate 2 is measured. Figure 8b shows the result of such a measurement which was done simultaneously with the measurement of the acceleration of the glass plate shown in Figure 8a. It can be seen that the same second vibrations appear in Figure 8b but with a smaller amplitude. In this test, the acceleration of the substrate 2, which was a painted metal plate, was measured in the direction of this plate 2. In an alternative embodiment, one could however also measure the acceleration of the substrate plate 2 in a direction forming an angle with the surface of this plate 2, in particular an angle larger than 45° and preferably an angle larger than 75°. Measuring the acceleration of the glass plate is however to be preferred since the amplitude of the vibrations of the substrate plate is influenced to a greater extend than the vibrations of the glass plate by the pressure exerted thereon by the gasket. From Figures 8a and 8b, it will further be clear that the signal of the second accelerometer can be used to filter, as explained hereinabove, the first vibration out of the acceleration values measured by the first accelerometer on the glass plate 17 and thus to show the second vibrations more clearly in the output signal.

From the above description, it will be clear that many modifications can be applied to the described method and apparatus provided they do not depart from the scope of protection as defined by the claims.

It is in particular possible to subject the glass plate with the gasket to the first vibration to obtain the relative movement between gasket and substrate or to vibrate both the glass plate and the substrate.

## Claims

1. A test method for assessing whether an assembly, which comprises as components at least a gasket and a substrate against which the gasket is pressed, has a tendency to squeak when the gasket is moved relative to the substrate, characterised in that for assessing said tendency, use is made of at least one accelerometer to detect whether vibrations are generated in the assembly when the gasket is moved relative to the substrate.

2. A method according to claim 1, characterised in that it comprises the steps of:
- pressing at least a piece of the gasket against at least a piece of the substrate;
- imposing a first vibration on at least one assembly component to obtain a relative motion between the gasket and the substrate;
- measuring the acceleration of at least one assembly component as a function of time during said first vibration; and
- generating an output signal which indicates said tendency to squeak in the case that, based on the measured acceleration values, said assembly component is subjected to at least one second vibration, having a frequency higher than the frequency of the first vibration.

3. A method according to claim 2, characterised in that said second vibration has a frequency higher than 100 Hz.

4. A method according to claim 2 or 3, characterised in that said output signal comprises a graph showing the acceleration values of at least said second vibration as a function of time.

5. A method according to any one of the claims 2 to 4, characterised in that said output signal comprises a value indicating an amplitude of the acceleration of said second vibration.

6. A method according to claim 5, characterised in that said acceleration amplitude indicating value is determined by determining the amplitudes of a number of successive peaks in the acceleration values of the second vibration and by selecting the maximum amplitude and/or by making an average of the determined amplitudes.

7. A method according to claim 5, characterised in that said acceleration amplitude indicating value is determined as an integrated surface area over a predetermined time period of the measured function between the acceleration of the second vibration and the time.

8. A method according to claim 5, characterised in that the measured function between the acceleration of the second vibration and the time is subjected to a Fourrier transformation and said acceleration amplitude indicating value is determined on the basis of this transformed function.

9. A method according to any one of the claims 5 to 8, characterised in that said acceleration amplitude indicating value is compared with a threshold acceleration indicating value pre-established for the second vibration and an acceleration amplitude indicating value lower than or equal to said pre-established threshold acceleration indicating value is correlated with a low tendency to squeak and an acceleration amplitude indicating value greater than said pre-established threshold acceleration indicating value is correlated with a too high tendency to squeak.

10. A method according to any one of the claims 2 to 9, characterised in that said assembly comprises as further component a plate onto which the gasket is applied and the acceleration of the gasket and/or of said plate is measured in a direction forming an angle with the surface of this plate, in particularly an angle larger than 45° and preferably an angle larger than 75°.

11. A method according to claim 10, characterised in that said first vibration is imposed on the substrate in a direction forming an angle with said plate, in particular an angle larger than 45°.

12. A method according to any one of the claims 2 to 11, characterised in that said substrate is in the form of a further plate and the acceleration of the substrate is measured, either in a direction parallel to the surface of this further plate or in a direction forming an angle with the surface of this further plate, in particular an angle larger than 45° and preferably an angle larger than 75°.

13. An apparatus for performing a test method according to any one of the claims 1 to 12, i.e. an apparatus for assessing whether an assembly, which comprises as components at least a gasket and a substrate against which the gasket is pressed, has a tendency to squeak when the gasket is moved relative to the substrate, which apparatus comprises first means for holding at least a piece of the gasket and second means for holding at least a piece of the substrate, the first and second holding means being arranged to press the piece of the gasket against the piece of the substrate, and means for moving the gasket relative to the substrate when being pressed against one another, characterised in that the apparatus further comprises at least one accelerometer for measuring the acceleration of at least one assembly component as a function of time during said first vibration.

14. An apparatus according to claim 11, characterised in that said means for moving the gasket relative to the substrate comprise at least one mechanical oscillator arranged to impose a first vibration on at least one assembly component to obtain said relative motion between the gasket and the substrate.

15. An apparatus according to claim 13 or 14, characterised in that it comprises means for generating an output signal which indicates said tendency to squeak in the case that, based on the measured acceleration values, said assembly component is subjected to at least one second vibration, having a frequency higher than at least 100 Hz.

16. An apparatus according to claim 15, characterised in that said output signal generating means are arranged to generate, as output signal, a graph showing the acceleration values of at least said second vibration as a function of time.

17. An apparatus according to claim 15 or 16, characterised in that said output signal generating means are arranged to generate, as output signal, a value indicating an amplitude of the acceleration of said second vibration.

18. An apparatus according to any one of the claims 15 to 17, characterised in that said output signal generating means are arranged to perform a Fourrier transformation on the function between the measured acceleration values and the time and to generate, as output signal, a further graph showing the transformed function and/or, a value deduced from this transformed function and indicating an amplitude of the acceleration of said second vibration.
